# EUROPEAN PATENT SPECIFICATION

(11) **EP 2 674 420 B1**
(45) Date of publication and mention of the grant of the patent: **27.01.2016**
(21) Application number: 12742587.4
(22) Date of filing: 02.02.2012
(51) Int. Cl.: C07D 239/52

(54) **METHOD FOR PRODUCING AMINOPHENYL PYRIMIDINYL ALCOHOL DERIVATIVE, AND SYNTHESIS INTERMEDIATE THEREOF**
VERFAHREN ZUR HERSTELLUNG EINES AMINOPHENYL-PYRIMIDINYL-ALKOHOLDERIVATS UND SYNTHESEZWISCHENPRODUKT DAFÜR
PROCÉDÉ DE PRODUCTION DE DÉRIVÉ D'ALCOOL AMINOPHÉNYLPYRIMIDINYLMÉTHYLIQUE ET INTERMÉDIAIRE DE SYNTHÈSE CORRESPONDANT

(30) Priority: 04.02.2011 JP 2011022868
(43) Date of publication of application: 18.12.2013
(73) Proprietor: Ihara Chemical Industry Co., Ltd., Tokyo 110-0008 (JP)
(72) Inventor: HAMADA, Yusuke, Fuji-shi, Shizuoka 421-3306 (JP); IKUMI, Akiko, Fuji-shi, Shizuoka 421-3306 (JP)
(74) Representative: Vossius & Partner Patentanwälte Rechtsanwälte mbB
(86) International application number: PCT/JP2012/000702
(87) International publication number: WO 2012/105263

(56) References cited:
- WO-A1-00/06553
- JP-A- H1 160 562
- JP-A- 11 060 562
- JP-A- 2003 212 861
- JP-A- 2006 056 870

## Description

### Technical Field

The present invention relates to a method for producing an aminophenylpyrimidinyl alcohol derivative that can be implemented with a simple operation under mild conditions without using a special reaction apparatus, and an intermediate for the synthesis thereof.

### Background Art

### (2-amino-3-methoxymethylphenyl)(4,6-dimethoxypyrimidin-2-yl)methanol,

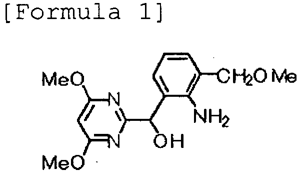

which is an aminophenylpyrimidinyl alcohol derivative, is known to be an intermediate for the synthesis of a herbicide (see Patent Literatures 1, 2, and 3).

As a method for obtaining above-mentioned (2-amino-3-methoxymethylphenyl)(4,6-dimethoxypyrimidin-2-yl)methanol, a method of reducing the nitro group of (4,6-dimethoxypyrimidin-2-yl)(3-methoxymethyl-2-nitrophenyl)ketone with an iron powder in the presence of acetic acid as a catalyst to form (2-amino-3-methoxymethylphenyl)(4,6-dimethoxypyrimidin-2-yl)ketone, and then reducing the carbonyl group with a metal hydride complex compound is known (see Patent Literatures 1 and 3).

However, this method uses, according to Examples in the Patent Literature 3, for example, a large amount of an iron powder which is not economical, and increases waste. Further, it also uses acetic acid, and therefore, acidic wastewater is generated. Accordingly, this method has a room for improvement in terms of economy, environment, operation, and the like.

As another method for obtaining (2-amino-3-methoxymethylphenyl)(4,6-dimethoxypyrimidin-2-yl)methanol, a method of reducing three functional groups, i.e., the chlorine atom, nitro group, and carbonyl group of (5-chloro-3-methoxymethyl-2-nitrophenyl)(4,6-dimethoxypyrimidin-2-yl)ketone, in one step in the same system by a catalytic hydrogenation method is also known (see Patent Literature 2). However, this method requires a hydrogen pressure as high as about 2 MPa and therefore is not preferable.

On the other hand, , as a method for producing an intermediate [VIIb] [i.e., an aminophenylpyrimidinyl alcohol derivative typified by (2-aminophenyl)(4,6-dimethoxypyrimidin-2-yl)methanol], from an intermediate [Va] [i.e., a nitrophenyl pyrimidinyl ketone derivative typified by (4,6-dimethoxypyrimidin-2-yl)(2-nitrophenyl)ketone], following routs are described by general formulas in a reaction scheme (Formula 9) in the Patent Literature 3, p. 41,
(1) a two-step route via an intermediate [Vb] [i.e., an aminophenyl pyrimidinyl ketone derivative typified by (2-aminophenyl)(4,6-dimethoxypyrimidin-2-yl)ketone], by reducing, first, the nitro group of the intermediate [Va] (i.e., a nitrophenyl pyrimidinyl ketone derivative), and
(2) a two-step route via an intermediate [VIIa] [i.e., a nitrophenylpyrimidinyl alcohol derivative typified by (2-nitrophenyl)(4,6-dimethoxypyrimidin-2-yl)methanol], by reducing, first, the carbonyl group of the intermediate [Va] (i.e., a nitrophenyl pyrimidinyl ketone derivative).

Here, as the method for producing (2-amino-3-alkoxymethylphenyl)(4,6-dimethoxypyrimidin-2-yl)methanol, the above route (1) via an intermediate [Vb] (i.e., an aminophenyl pyrimidinyl ketone derivative) which involves reducing the nitro group of the intermediate [Va] first, is specifically described not only in Reference Example 3, Patent Literature 3, but also in (4) and (5), Reference Example 2, Patent Literature 1. However, the above route (2) via an intermediate [VIIa] (i.e., a nitrophenylpyrimidinyl alcohol derivative) which involves reducing the carbonyl group of the intermediate [Va] first, is not specifically described. Further, there is no specific description of (3-alkoxymethyl-2-nitrophenyl)(4,6-dimethoxypyrimidin-2-yl)methanol, and the industrial advantages of the route via the intermediate [VIIa] are not even suggested.

### Prior Art Literatures

### Patent Literature

### Patent Literature 1: WO 00/06553

Patent Literature 2: Japanese Patent Application Laid-Open No. 2003-212861
Patent Literature 3: Japanese Patent Application Laid-Open No. Hei-11-60562

### Summary of Invention

### Task to Be Achieved by the Invention

It is an object of the present invention to provide a method for producing an aminophenylpyrimidinyl alcohol derivative typified by (2-amino-3-methoxymethylphenyl)(4,6-dimethoxypyrimidin-2-yl)methanol from a nitrophenyl pyrimidinyl ketone derivative typified by (4,6-dimethoxypyrimidin-2-yl)(3-methoxymethyl-2-nitrophenyl)ketone that can solve the drawbacks in the prior art described above.

It is another object of the present invention to provide a method for producing the aminophenylpyrimidinyl alcohol derivative that has good efficiency and can be implemented on an industrial scale with a simple operation; is industrially preferable because of high yield; and further can reduce environmental load.

It is a further object of the present invention to provide an industrially preferable method for producing the aminophenylpyrimidinyl alcohol derivative that can be implemented under mild conditions without using a special reaction apparatus, by using an appropriate amount of an iron powder as a reducing agent, which is easily available.

It is a further object of the present invention to provide an intermediate useful in the above methods for producing the aminophenylpyrimidinyl alcohol derivative.

### Means for Achieving the Task

In view of the circumstances as described above, the present inventors have repeatedly and diligently studied methods for producing (2-amino-3-alkoxymethylphenyl)(4,6-dimethoxypyrimidin-2-yl)methanol, and, as a result, unexpectedly found that the above problems can be solved by way of a nitrophenylpyrimidinyl alcohol derivative represented by the following general formula (2), 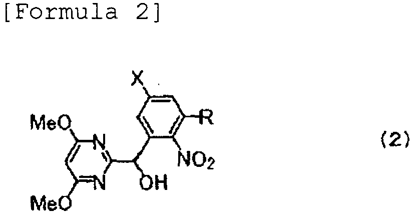 that is, by changing the order of the reduction of the carbonyl group and the nitro group in the production method specifically described in the Reference Examples in the Patent Literature 1 and Patent Literature 3, and by simultaneously providing a nitrophenylpyrimidinyl alcohol derivative represented by general formula (2). Based on this finding, the present inventors have completed the present invention.

Specifically, the present invention solves the above problems by providing inventions according to the following items [1] to [12].

[1] A method for producing an aminophenylpyrimidinyl alcohol derivative represented by general formula (3): 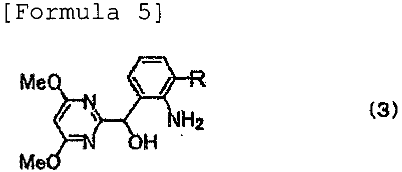 wherein R is an alkoxymethyl group,
   comprising reducing a carbonyl group of a nitrophenyl pyrimidinyl ketone derivative represented by general formula (1): 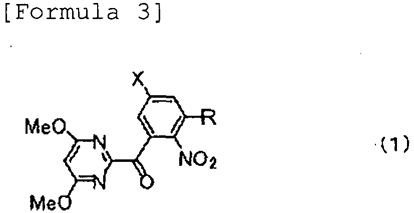 wherein X is a hydrogen atom, and R is the same as described above,
   to produce a nitrophenylpyrimidinyl alcohol derivative represented by general formula (2): 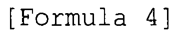 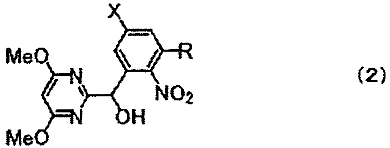 wherein X and R are the same as described above,
   and then reducing the alcohol derivative using an iron powder in the presence of a salt selected from ammonium salts and alkaline-earth metal salts, without adding a free acid.
[2] The method for producing an aminophenylpyrimidinyl alcohol derivative according to [1], wherein the nitrophenyl pyrimidinyl ketone derivative represented by general formula (1) is reduced using a metal hydride.
[3] The method for producing an aminophenylpyrimidinyl alcohol derivative according to [2], wherein the nitrophenyl pyrimidinyl ketone derivative represented by general formula (1) is reduced using sodium borohydride.
[4] The method for producing an aminophenylpyrimidinyl alcohol derivative according to [1], wherein the nitrophenylpyrimidinyl alcohol derivative represented by general formula (2) is reduced in the presence of an ammonium halide salt, an ammonium carbonate salt, an ammonium carboxylate salt, an alkaline-earth metal halide salt, or an alkaline-earth metal carbonate salt.
[5] The method for producing an aminophenylpyrimidinyl alcohol derivative according to [1], wherein the nitrophenylpyrimidinyl alcohol derivative represented by general formula (2) is reduced in the presence of an ammonium halide salt, an ammonium carboxylate salt, or an alkaline-earth metal halide salt.
[6] The method for producing an aminophenylpyrimidinyl alcohol derivative according to [1], wherein the nitrophenylpyrimidinyl alcohol derivative represented by general formula (2) is reduced in the presence of ammonium chloride, ammonium acetate, calcium chloride, or barium chloride.
[7] The method for producing an aminophenylpyrimidinyl alcohol derivative according to [1], wherein the nitrophenylpyrimidinyl alcohol derivative represented by general formula (2) is reduced in the presence of ammonium chloride or ammonium acetate.
[8] The method for producing an aminophenylpyrimidinyl alcohol derivative according to [1], wherein the nitrophenylpyrimidinyl alcohol derivative represented by general formula (2) is reduced in the presence of ammonium chloride.
[9] The method for producing an aminophenylpyrimidinyl alcohol derivative according to [1], wherein the nitrophenylpyrimidinyl alcohol derivative represented by general formula (2) is reduced in the presence of ammonium acetate.
[10] The method for producing an aminophenylpyrimidinyl alcohol derivative according to any one of [1] to [9], wherein the nitrophenylpyrimidinyl alcohol derivative represented by general formula (2) is reduced using 2.0 to 3.0 moles of an iron powder with respect to 1 mole of the nitrophenylpyrimidinyl alcohol derivative represented by general formula (2).
[11] The method for producing an aminophenylpyrimidinyl alcohol derivative according to any one of [1] to [10], wherein R is a methoxymethyl group.
[12] A nitrophenylpyrimidinyl alcohol derivative represented by general formula (2): 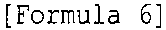 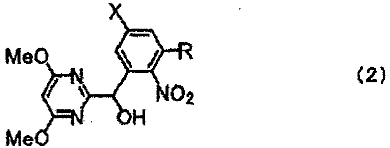 wherein X is a hydrogen atom, and R is a methoxymethyl group.

### Advantageous Effects of Invention

The present invention provides a novel industrial production method for an aminophenylpyrimidinyl alcohol derivative represented by general formula (3), and a nitrophenylpyrimidinyl alcohol derivative represented by general formula (2).

According to the method of the present invention, where X is a hydrogen atom in the nitrophenyl pyrimidinyl ketone derivative represented by general formula (1), by reducing its carbonyl group first, the following reduction of the nitro group can be performed using, as a reducing agent, an easily available iron powder in an almost theoretical amount, rather than in large excess as in the reaction described in the Examples in the Patent Literature 1, and the target aminophenylpyrimidinyl alcohol derivative represented by general formula (3) can be efficiently produced with a simple operation with high yield under the mild conditions of not using high pressure hydrogen, without requiring a special reaction apparatus. Further, by reducing the amount of waste derived from the above reducing agent, the environmental load can be reduced.

The above reduction of the nitro group does not require acetic acid used in the reaction described in the Patent Literature 1, and can be performed without adding a free acid, that is, under substantially neutral reaction conditions. Also by making the properties of waste preferable, the environmental load can be reduced. In the route via the intermediate [Vb] (i.e., an aminophenyl pyrimidinyl ketone derivative) produced by reducing the nitro group first, specifically described in the Patent Literature 1 and Patent Literature 3 as Reference Example, when the reduction of the nitro group was performed without adding a free acid typified by a carboxylic acid, such as acetic acid, that is, under substantially neutral conditions, , the reaction did not proceed sufficiently, as shown in Comparative Example 1 described later.

Therefore, by changing the order of the reduction of the carbonyl group and the nitro group in the production method specifically described in the Reference Examples in the Patent Literature 1 and Patent Literature 3, and by simultaneously providing a nitrophenylpyrimidinyl alcohol derivative represented by general formula (2), a plurality of effects with different natures as described above were obtained, and a plurality of drawbacks were simultaneously solved.

### Best Mode for Carrying out the Invention

The present invention is described in detail below.

The present invention relates to a method for producing an aminophenylpyrimidinyl alcohol derivative, comprising reducing the carbonyl group of a nitrophenyl pyrimidinyl ketone derivative represented by general formula (1), first, to produce a nitrophenylpyrimidinyl alcohol derivative represented by general formula (2) and then reducing this derivative, and a nitrophenylpyrimidinyl alcohol derivative represented by general formula (2), which is a useful intermediate.

### {Nitrophenyl Pyrimidinyl Ketone Derivative Represented by general formula (1)}

First, the nitrophenyl pyrimidinyl ketone derivative represented by general formula (1), used as a raw material in the production method of the present invention, is described.

The substituent X in general formula (1) is a hydrogen atom.

The group R in general formula (1) is an alkoxymethyl group.

An alkoxymethyl group is, for example, a (straight chain or branched chain C1 to C4 alkoxy)-methyl group. "C1 to C4" means that the number of carbon atoms of a substituent following this term is 1 to 4. For example, a (straight chain or branched chain C1 to C4 alkoxy)-methyl group is a methoxymethyl group, an ethoxymethyl group, a propoxymethyl group, an isopropoxymethyl group, a butoxymethyl group, an isobutoxymethyl group, a sec-butoxymethyl group, or a tert-butoxymethyl group.

Therefore, specific examples of the nitrophenyl pyrimidinyl ketone derivative represented by general formula (1) include;
(4,6-dimethoxypyrimidin-2-yl) (3-methoxymethyl-2-nitrophenyl)ketone,
(4,6-dimethoxypyrimidin-2-yl) (3-ethoxymethyl-2-nitrophenyl)ketone,
(4,6-dimethoxypyrimidin-2-yl) (3-propoxymethyl-2-nitrophenyl)ketone,
(4,6-dimethoxypyrimidin-2-yl) (3-isopropoxymethyl-2-nitrophenyl)ketone.

The nitrophenyl pyrimidinyl ketone derivative represented by general formula (1) is a publicly known compound, or a compound that can be produced, for example, by the methods described in the Patent Literatures 1, 2, and 3.

### {Method for Producing Nitrophenylpyrimidinyl Alcohol Derivative Represented by general Formula (2)}

Next, the step for producing the nitrophenylpyrimidinyl alcohol derivative represented by general formula (2) by reducing the carbonyl group of the nitrophenyl pyrimidinyl ketone derivative represented by general formula (1) is described.

### (Reducing Agent)

Any reducing agent may be used in the reaction in this step as long as the carbonyl group of the nitrophenyl pyrimidinyl ketone derivative represented by general formula (1) can be reduced. Specifically, metal hydrides can be mentioned as examples, but the reducing agent is not limited to these.

### (Metal Hydrides)

Specific examples of metal hydrides that can be used as the reducing agent in this reaction include, but are not limited to, boron hydride compounds, such as sodium borohydride, sodium cyanoborohydride, sodium triacetoxyborohydride, lithium tri(sec-butyl)borohydride, calcium borohydride, or a borane-tetrahydrofuran complex; aluminum hydride compounds, such as lithium aluminum hydride or diisobutylaluminum hydride; silicon hydride compounds, such as triethylsilane or trichlorosilane and the like. The reducing agents, such as these metal hydrides, may be used singly, or as a mixture of two or more kind in any ratio.

From the viewpoints of availability, ease of handling, reactivity, price, and the like, the use of sodium borohydride, sodium cyanoborohydride, sodium triacetoxyborohydride, calcium borohydride, a borane-tetrahydrofuran complex, or lithium aluminum hydride is preferable; the use of sodium borohydride or lithium aluminum hydride is more preferable; and the use of sodium borohydride is further preferable.

### (Amount of Reducing Agent Used, Such As Metal Hydride)

The molar ratio of the reducing agent in this reaction may be in any range as long as it allows sufficient progress of the reaction of the nitrophenyl pyrimidinyl ketone derivative represented by general formula (1) (raw material compound) However, the range of generally 0.1 to 10.0 moles, preferably 0.2 to 10.0 moles, more preferably 0.25 to 5 moles, further preferably 0.25 to 3.0 moles, and particularly preferably 0.25 to 1.5 moles, with respect to 1 mole of the nitrophenyl pyrimidinyl ketone derivative represented by general formula (1) (raw material compound), can be mentioned as examples.

### (Alcohol)

In this reaction, when a boron hydride compound is used, use of an alcohol as well is preferable. Specific examples of the alcohol used in this reaction include C1 to C6 alkanols typified by methanol, ethanol, propanol, isopropanol, and butanol; C1 to C6 alkanediols typified by ethylene glycol and the like. These alcohols may be used singly or mixed in any ratio. From the viewpoints of availability, ease of handling, reactivity, and the like, the use of methanol, ethanol is preferable, and the use of methanol is more preferable.

### (Amount of Alcohol Used)

The molar ratio of the alcohol in this reaction may be in any range as long as the reaction proceeds sufficiently. However, the range of generally 0.01 moles or more, preferably 0.1 to 20.0 moles, more preferably 0.1 to 10.0 moles, and further preferably 0.1 to 5.0 moles, with respect to the nitrophenyl pyrimidinyl ketone derivative represented by general formula (1) (raw material compound), can be mentioned as examples. Incidentally, when the alcohol is also used as a solvent described later, a large excess amount of the alcohol may be used regardless of the range mentioned here as examples.

### (Solvent)

This reaction can also be carried out without a solvent. However, in order to allow the reaction to proceed smoothly, a solvent is preferably used.

The solvent that can be used in this reaction may be any solvent as long as it does not inhibit the reaction. Examples of the solvent include, but are not limited to, aprotic polar solvents, such as N,N-dimethylformamide (DMF), N,N-dimethylacetamide (DMAC), acetonitrile, or propylene carbonate; ethers, such as diphenyl ether, diethyl ether, tetrahydrofuran (THF), or dioxane; aromatic hydrocarbons, such as toluene, xylene, chlorobenzene, or dichlorobenzene; aliphatic hydrocarbons, such as pentane or hexane; halogen-containing hydrocarbons, such as dichloromethane; alcohols, such as methanol, ethanol, propanol, isopropanol, butanol, or ethylene glycol; water and the like.

Especially, as the solvent, ethers, such as diphenyl ether, diethyl ether, diisopropyl ether, tetrahydrofuran (THF), or dioxane; aromatic hydrocarbons, such as toluene, xylene, chlorobenzene, or dichlorobenzene; alcohols, such as methanol, ethanol, propanol, isopropanol, butanol, or ethylene glycol; and water are preferable, and aromatic hydrocarbons, such as toluene, xylene, chlorobenzene, or dichlorobenzene; and alcohols, such as methanol, ethanol, propanol, isopropanol, butanol, or ethylene glycol, are further preferable, and toluene or methanol is particularly preferable. The solvent can be used singly or as a mixed solvent in any mixing ratio.

### (Amount of Solvent)

The amount of the solvent may be any amount as long as it allows for sufficient stirring of the reaction system. However, the range of generally 0 to 20 L, preferably 0.1 to 10 L, more preferably 0.3 to 2.0 L, and further preferably 0.5 to 1.5 L, with respect to 1 mole of the nitrophenyl pyrimidinyl ketone derivative represented by general formula (1) (raw material compound), can be mentioned as examples.

### (Reaction Temperature)

As the reaction temperature of this reaction, for example, the range of -10°C to the reflux temperature of the solvent used can be mentioned. The range of -10°C to 30°C is preferable, the range of -10°C to 20°C is more preferable, and the range of -5°C to 10°C is further preferable.

### (Reaction Time)

The reaction time of this reaction is not particularly limited. From the viewpoint of by-product suppression, and the like, 1 hour to 48 hours can be preferably mentioned, and 1 hour to 30 hours can be further preferably mentioned, for example.

### (Nitrophenylpyrimidinyl Alcohol Derivative Represented by general formula (2))

Next, the nitrophenylpyrimidinyl alcohol derivative represented by general formula (2), produced from the nitrophenyl pyrimidinyl ketone derivative represented by general formula (1) as described above, is described.

X and R in the nitrophenylpyrimidinyl alcohol derivative represented by general formula (2) are the same as in the above nitrophenyl pyrimidinyl ketone derivative represented by general formula (1).

Specific examples of the nitrophenylpyrimidinyl alcohol derivative represented by general formula (2) include;
(4,6-dimethoxypyrimidin-2-yl) (3-methoxymethyl-2-nitrophenyl)methanol,
(4,6-dimethoxypyrimidin-2-yl). (3-ethoxymethyl-2-nitrophenyl)methanol,
(4,6-dimethoxypyrimidin-2-yl) (2-nitro-3-propoxymethylphenyl)methanol,
(4,6-dimethoxypyrimidin-2-yl) (3-isopropoxymethyl-2-nitrophenyl)methanol.

### {Method for Producing Aminophenylpyrimidinyl Alcohol Derivative Represented by general formula (3)}

Next, the step for producing the aminophenylpyrimidinyl alcohol derivative represented by general formula (3) by reducing the nitrophenylpyrimidinyl alcohol derivative represented by general formula (2) is described.

### (Reduction Reaction)

As reduction reaction in this step, a reduction reaction using an iron powder as a reducing agent is used, because a special reaction apparatus, high pressure hydrogen, and the like are not required.

### (Reduction Reaction Using Metal as Reducing Agent)

### (Metal)

In a reduction reaction, iron powder is used from the viewpoints of availability, ease of handling, reactivity, price, and the like.

### (Amount of Iron Powder Used)

The amount of the iron powder used as the reducing agent in this reaction may be any amount as long as the reaction proceeds sufficiently. However, the range of generally 0.5 to 50.0 moles, preferably 0.5 to 20.0 moles, more preferably 2.0 to 5.0 moles, further preferably 2.0 to 4.0 moles, and particularly preferably 2.0 to 3.0 moles, with respect to 1 mole of the nitrophenylpyrimidinyl alcohol derivative represented by general formula (2) (raw material compound), can be mentioned as examples. The theoretical amount of the metal as the reducing agent is 2 equivalents, and by using an almost the theoretical amount of the reducing agent, the amount of waste derived from the reducing agent can be reduced.

### (Salt)

In the above reduction reaction using an iron powder as a reducing agent, a salt selected from ammonium salts and alkaline-earth metal salts is used.

As the anions forming the salts, anions derived from acids can be mentioned as examples, but the anions are not limited to these ones.

As acids in the above anions, inorganic acids, such as hydrohalic acids typified by hydrochloric acid, hydrobromic acid, hydrofluoric acid, sulfuric acid, nitric acid, or phosphoric acid; carboxylic acids, such as formic acid, acetic acid, propionic acid, trifluoroacetic acid, or benzoic acid ; sulfonic acids, such as methanesulfonic acid, trifluoromethanesulfonic acid, benzenesulfonic acid, or p-toluenesulfonic acid, or the like can be mentioned as examples, but the acids are not limited to these ones.

As the above salts, specifically, ammonium halide salts, such as ammonium chloride or ammonium bromide; alkaline-earth metal halide salts, such as magnesium chloride, barium chloride, or calcium chloride; ammonium carbonate salts, such as ammonium carbonate or ammonium hydrogen carbonate; alkaline-earth metal carbonate salts, such as magnesium carbonate, barium carbonate, calcium carbonate, or calcium hydrogen carbonate; ammonium sulfate salts, such as ammonium sulfate or ammonium hydrogen sulfate; alkaline-earth metal sulfate salts, such as barium sulfate, calcium sulfate, or calcium hydrogen sulfate; ammonium nitrate; ammonium carboxylate salts, such as ammonium formate, ammonium acetate, ammonium propionate, ammonium trifluoroacetate, or ammonium benzoate; alkaline-earth metal carboxylate salts, such as magnesium acetate, magnesium propionate, calcium acetate, or calcium propionate; ammonium sulfonate salts, such as ammonium benzenesulfonate; or alkaline-earth metal sulfonate salts, such as barium benzenesulfonate, can be mentioned as examples, but the salts are not limited to these ones.

From the viewpoints of availability, ease of handling, reactivity, price, and the like, the use of an ammonium halide salt, an ammonium carbonate salt, an ammonium carboxylate salt, an alkaline-earth metal halide salt, or an alkaline-earth metal carbonate salt is preferable; the use of an ammonium halide salt, an ammonium carboxylate salt, or an alkaline-earth metal halide salt is further preferable; the use of ammonium chloride, ammonium acetate, calcium chloride, or barium chloride is further preferable; and the use of ammonium chloride or ammonium acetate is particularly preferable.

These salts may be anhydrides or hydrates, and these salts may be simple salts or double salts. In addition, these salts may be used singly, or as a mixture of two or more kind in any ratio.

### (Amount of Salt Used)

The molar ratio of the salt in this reaction may be in any range as long as the reaction proceeds sufficiently. However, the range of generally 0.01 to 50.0 moles, preferably 0.01 to 10.0 moles, more preferably 0.05 to 5.0 moles, further preferably 0.1 to 3.0 moles, and particularly preferably 0.1 to 1.0 moles, with respect to 1 mole of the nitrophenylpyrimidinyl alcohol derivative represented by general formula (2) (raw material compound), can be mentioned as examples.

### (Free Acid)

### (Free Carboxylic Acid)

In the reduction reaction using an iron powder as a reducing agent, the reduction of the nitro group proceeds sufficiently without adding an acid typified by a free carboxylic acid, such as acetic acid, unlike the prior art, such as the Patent Literature 1. In other words, the reduction of the nitro group proceeds sufficiently under substantially neutral reaction conditions. Therefore, it is not necessary to discharge an acidic waste liquid and neutralize it, and accordingly, the environmental load can be reduced.

### (Water or Alcohols)

Water or alcohols are preferably used in the reduction reaction using an iron powder as a reducing agent. As the alcohols that can be used in this reaction, for example, alcohols described later as solvents that can be used in this reaction, can be mentioned.

### (Amount of Water or Alcohols Used)

The molar ratio of the water or alcohols used in this reaction may be in any range as long as the reaction proceeds sufficiently. However, the range of generally 0 moles or more, preferably 0.01 moles or more, more preferably 0.01 to 2000 moles, further preferably 1 to 100 moles, and particularly preferably 5 to 20 moles, with respect to the nitrophenylpyrimidinyl alcohol derivative represented by general formula (2) (raw material compound), can be mentioned as examples. Incidentally, when the water or alcohols are also used as a solvent described later, a large excess amount of the water or alcohols may be used regardless of the range aforementioned as examples.

### (Solvent)

The reaction using an iron powder as a reducing agent can also be carried out without a solvent. However, in order to allow the reaction to proceed smoothly, a solvent is preferably used.

The solvent that can be used in this reaction may be any solvent as long as it does not inhibit the reaction. Examples of the solvent include, but are not limited to, aprotic polar solvents, such as N,N-dimethylformamide (DMF), N,N-dimethylacetamide (DMAC), acetonitrile, or propylene carbonate; ethers, such as diphenyl ether, diethyl ether, diisopropyl ether, tetrahydrofuran (THF), or dioxane; aromatic hydrocarbons, such as toluene or xylene; aliphatic hydrocarbons, such as pentane or hexane; halogen-containing hydrocarbons, such as dichloromethane, chlorobenzene, or dichlorobenzene; alcohols, such as methanol, ethanol, propanol, isopropanol, butanol, or ethylene glycol; and water and the like. Especially, as the solvent, aromatic hydrocarbons (such as toluene or xylene), alcohols (such as methanol, ethanol, propanol, isopropanol, butanol, or ethylene glycol), or water is preferable, and toluene, xylene, methanol, ethanol, isopropanol, or water is further preferable. The solvent can be used singly or as a mixed solvent in any mixing ratio.

### (Amount of Solvent)

The amount of the solvent may be any amount as long as it allows sufficient stirring of the reaction system. However, the range of generally 0 to 10 L, preferably 0.05 to 10 L, more preferably 0.1 to 5 L, further preferably 0.2 to 2 L, and particularly preferably 0.2 to 1.5 L, with respect to 1 mole of the nitrophenylpyrimidinyl alcohol derivative represented by general formula (2) (raw material compound), can be mentioned as examples.

### (Reaction Temperature)

As the reaction temperature of this reaction, for example, the range of 0°C to the reflux temperature of the solvent used can generally be mentioned. The range of preferably 0°C to 150°C, more preferably 20°C to 100°C, further preferably 40°C to 100°C, and particularly preferably 60 to 100°C can be mentioned as examples.

### (Reaction Time)

The reaction time of this reaction is not particularly limited. However, from the viewpoint of by-product suppression, and the like, 1 hour to 48 hours can be preferably mentioned, and 1 hour to 30 hours can be further preferably mentioned, for example.

### (Aminophenylpyrimidinyl Alcohol Derivative Represented by general formula (3))

Next, the aminophenylpyrimidinyl alcohol derivative represented by general formula (3), obtained by the method of the present invention, is described.

X and R in the aminophenylpyrimidinyl alcohol derivative represented by general formula (3) are the same as in the above nitrophenyl pyrimidinyl ketone derivative represented by general formula (1).

Specific examples of the aminophenylpyrimidinyl alcohol derivative represented by general formula (3) can include;
(2-amino-3-methoxymethylphenyl)(4,6-dimethoxypyrimidin-2-yl)methanol,
(2-amino-3-ethoxymethylphenyl)(4,6-dimethoxypyrimidin-2-yl)methanol,
(2-amino-3-propoxymethylphenyl)(4,6-dimethoxypyrimidin-2-yl)methanol,
(2-amino-3-isopropoxymethylphenyl)(4,6-dimethoxypyrimidin-2-yl)methanol,
(2-amino-3-butoxymethylphenyl)(4,6-dimethoxypyrimidin-2-yl)methanol,
(2-amino-3-isobutoxymethylphenyl)(4,6-dimethoxypyrimidin-2-yl)methanol,
(2-amino-3-sec-butoxymethylphenyl)(4,6-dimethoxypyrimidin-2-yl)methanol, and
(2-amino-3-tert-butoxymethylphenyl)(4,6-dimethoxypyrimidin-2-yl)methanol.

### Examples

Next, the methods for producing the compounds of the present invention is specifically described by giving Examples, however the present invention is not limited in any way by these Examples.

### Example 1

### Production of (4,6-dimethoxypyrimidin-2-yl)(3-methoxymethyl-2-nitrophenyl)methanol {Compound of General Formula (2) wherein X = H and R = Methoxymethyl}: Reduction of Carbonyl Group with Sodium Borohydride

9.33 g (28.0 mmol) of (4,6-dimethoxypyrimidin-2-yl)(3-methoxymethyl-2-nitrophenyl)ketone, 28 ml of toluene, and 3.2 ml (78 mmol) of methanol were added to a 100 ml four-necked flask equipped with a stirrer, a reflux condenser, a thermometer, and a dropping funnel, and the mixture was cooled to 0 to 5°C with stirring, and 0.32 g (8.4 mmol) of sodium borohydride was added in a small portion. Then, the mixture was stirred at 0 to 5°C for 1 hour. 14.9 ml (8.4 mmol) of 2% hydrochloric acid was added dropwise into the reaction liquid, and the mixture was separated at 50°C. Then, the toluene layer was washed with 14 ml of water, and toluene was distilled off under reduced pressure. Then, purification was performed by recrystallization by adding 12 ml of isopropanol and 5 ml of water. (4,6-dimethoxypyrimidin-2-yl)(3-methoxymethyl-2-nitrophenyl)methanol was obtained as a whitish solid with a yield of 91%.
Melting point: 93.3°C
¹H-NMR (300 MHz, CDCl₃, σ) : 7.43 - 7.57 (m, 3H), 6.01 (d, J = 4.5 Hz, 1H), 5.87 (s, 1H) 4.91 (d, J = 4.5 Hz, 1H), 4.50 (s, 2H), 3.90 (s, 6H), 3.35 (s, 3H)
LC-MS (m/z): 336.1 [M+H]⁺

### Example 2

### Production of (2-amino-3-methoxymethylphenyl)(4,6-dimethoxypyrimidin-2-yl)methanol {Compound of General Formula (3) wherein X = H and R = Methoxymethyl}: Reduction of Nitro Group with Iron Powder

13.33 g (0.04 mol) of (4,6-dimethoxypyrimidin-2-yl)(3-methoxymethyl-2-nitrophenyl)methanol, 5.58 g (0.10 mol, 2.5 equivalents) of an iron powder, and 40 ml of ethanol were added to a 100 ml four-necked flask equipped with a stirrer, a reflux condenser, a thermometer, and a dropping funnel, and the mixture was heated to 80°C with stirring. A mixed liquid of 1.07 g (0.02 mol) of ammonium chloride in 12 ml of water was added dropwise therein, and the mixture was stirred at 80°C for 1 hour. The reaction liquid was cooled to 25°C, and then filtered using Celite. Ethanol was distilled off from the filtrate under reduced pressure, and then, 20 ml of chlorobenzene and 20 ml of water were added, and the mixture was stirred and separated. The chlorobenzene layer was washed with 40 ml of water to obtain (2-amino-3-methoxymethylphenyl)(4,6-dimethoxypyrimidin-2-yl)methanol as a yellow chlorobenzene solution. This solution was analyzed by an HPLC absolute calibration curve method, and as a result, the yield was 93%.

### Example 3

### (Example in Which Ammonium Acetate Was Used Instead of Ammonium Chloride in Example 2)

6.98 g (0.125 mol, 2.5 equivalents) of an iron powder, 1.93 g (0.025 mol) of ammonium acetate, and 15 ml of water were added to a 100 ml four-necked flask equipped with a stirrer, a reflux condenser, a thermometer, and a dropping funnel, and the mixture was heated to 80°C with stirring. A slurry of 39 g (0.05 mol) of (4,6-dimethoxypyrimidin-2-yl)(3-methoxymethyl-2-nitrophenyl)methanol and 50 ml of isopropanol was added dropwise therein, and the mixture was stirred at 80°C for 4 hours. The reaction liquid was cooled to 25°C, and then filtered using Celite. Isopropanol was distilled off from the filtrate under reduced pressure, and then, 25 ml of chlorobenzene was added, and the mixture was stirred and separated. The chlorobenzene layer was washed with 50 ml of water to obtain (2-amino-3-methoxymethylphenyl)(4,6-dimethoxypyrimidin-2-yl)methanol as a yellow chlorobenzene solution. This solution was analyzed by the HPLC absolute calibration curve method, and as a result, the yield was 91%.

### Example 4

### (Example in Which Ammonium Acetate Was Used Instead of Ammonium Chloride in Example 2)

121.45 g (2.17 mol, 2.5 equivalents) of an iron powder, 33.53 g (0.435 mol) of ammonium acetate, and 261 ml of water were added to a 1000 ml four-necked flask equipped with a stirrer, a reflux condenser, a thermometer, and a dropping funnel, and the mixture was heated to 80°C with stirring. A slurry of 291.71 g (0.87 mol) of (4,6-dimethoxypyrimidin-2-yl)(3-methoxymethyl-2-nitrophenyl)methanol and 292 ml of isopropanol was added dropwise therein, and the mixture was stirred at 80°C for 4 hours. The reaction liquid was cooled to 25°C, and then filtered using Celite. Isopropanol was distilled off from the filtrate under reduced pressure, and then, 435 ml of chlorobenzene was added, and the mixture was stirred and separated. The pH of the aqueous layer at this time was 7.9. The chlorobenzene layer was washed with 435 ml of water to obtain (2-amino-3-methoxymethylphenyl)(4,6-dimethoxypyrimidin-2-yl)methanol as a yellow chlorobenzene solution. This solution was analyzed by the HPLC absolute calibration curve method, and as a result, the yield was 95%.

The pH of the above aqueous phase, that is, wastewater, in Example 4 conducted without addition of a free acid is 7.9, which is not acidic, and therefore, neutralization is not particularly required. In addition, in the light of the compounds, reagents, and the like used, the liquid properties of the aqueous phase are also expected to be neutral in other Examples.

### Example 5

### (Example in Which Calcium Chloride Was Used Instead of Ammonium Chloride in Example 2.)

1.68 g (5 mmol) of (4,6-dimethoxypyrimidin-2-yl)(3-methoxymethyl-2-nitrophenyl)methanol, 0.7 g (13 mmol, 2.6 equivalents) of an iron powder, and 5 ml of ethanol were added to a 50 ml eggplant-shaped flask equipped with a magnet stirrer and a dropping funnel, and the mixture was heated to 80°C with stirring. 0.28 g (3 mmol) of calcium chloride and 1.5 ml of water were added dropwise therein, and the mixture was stirred at 80°C for 6 hours. The reaction liquid was analyzed by HPLC, and as a result, the yield of (2-amino-3-methoxymethylphenyl)(4,6-dimethoxypyrimidin-2-yl)methanol was 93.4% in terms of HPLC area percentage.

### Example 6

### (Example in Which Barium Chloride Was Used Instead of Ammonium Chloride in Example 2.)

1.68 g (5 mmol) of (4,6-dimethoxypyrimidin-2-yl)(3-methoxymethyl-2-nitrophenyl)methanol, 0.7 g (13 mmol, 2.6 equivalents) of an iron powder, and 5 ml of ethanol were added to a 50 ml eggplant-shaped flask equipped with a magnet stirrer and a dropping funnel, and the mixture was heated to 80°C with stirring. 0.52 g (3 mmol) of barium chloride and 1.5 ml of water were added dropwise therein, and the mixture was stirred at 80°C for 2 hours. The reaction liquid was analyzed by HPLC, and as a result, the yield of (2-amino-3-methoxymethylphenyl)(4,6-dimethoxypyrimidin-2-yl)methanol was 91.9% in terms of HPLC area percentage.

### Comparative Example 1

### {Example in Which (4,6-dimethoxypyrimidin-2-yl)(3-methoxymethyl-2-nitrophenyl)ketone, Rather Than (4,6-dimethoxypyrimidin-2-yl)(3-methoxymethyl-2-nitrophenyl)methanol, Was Used as Raw Material under Same Reaction Conditions As in Examples 3 and 4} Production of (2-amino-3-methoxymethylphenyl)(4,6-dimethoxypyrimidin-2-yl)ketone

0.7 g (12.5 mmol, 2.5 equivalents) of an iron powder, 0.193 g (2.5 mmol) of ammonium acetate, and 1.5 ml of water were added to a 100 ml four-necked flask equipped with a stirrer, a reflux condenser, a thermometer, and a dropping funnel, and the mixture was heated to 80°C with stirring. A slurry of 1.67 gg (5 mmol) of (4,6-dimethoxypyrimidin-2-yl)(3-methoxymethyl-2-nitrophenyl)ketone and 5 ml of isopropanol was added dropwise therein, and the mixture was heated and stirred at 80°C for 4 hours. The reaction liquid was analyzed by HPLC, and as a result, the target (2-amino-3-methoxymethylphenyl)(4,6-dimethoxypyrimidin-2-yl)ketone was not detected, and the raw material (4,6-dimethoxypyrimidin-2-yl)(3-methoxymethyl-2-nitrophenyl)ketone remained.

Further, the reaction liquid was heated and stirred at the same temperature for another 4 hours. The reaction liquid was analyzed by HPLC, and as a result, the target (2-amino-3-methoxymethylphenyl)(4,6-dimethoxypyrimidin-2-yl)ketone was produced in a small amount, as an HPLC area percentage of 32%. In addition, the raw material (4,6-dimethoxypyrimidin-2-yl)(3-methoxymethyl-2-nitrophenyl)ketone in an amount as large as an HPLC area percentage of 43% remained. Further, impurities in an amount as large as an HPLC area percentage of 25% were produced as by-products.

As described above, the reaction did not proceed with the same reaction time as in Examples 3 and 4, and therefore, the reaction time was extended to twice as long as that in Examples 3 and 4. Then, the reaction proceeded, however, only a significantly lower yield than the high yields in Examples 3 and 4 was obtained, and a large amount of impurities were produced as by-products.

### Comparative Example 2

### {Method Described in Reference Example 2 (4), WO 00/06553 (Patent Literature 1), p. 17}

### Production of (2-amino-3-methoxymethylphenyl)(4,6-dimethoxypyrimidin-2-yl)ketone: Reduction of Nitro Group of Ketone Derivative with Iron Powder

A mixture of 3.3 g (10 millimoles) of (4,6-dimethoxypyrimidin-2-yl)(3-methoxymethyl-2-nitrophenyl) ketone, 3 g (54 millimoles) of an iron powder, 20 ml of water, 150 ml of ethyl acetate, and 1 ml of acetic acid were subjected to a reaction at 50°C for 5 hours. The insolubles in the reaction liquid were filtered off using a filter aid, and the organic layer was washed with a saturated aqueous sodium chloride solution, and then dried. The solvent was distilled off under reduced pressure, and the crystalline residue was washed with diisopropyl ether to obtain 2.4 g (yield: 80%) of (2-amino-3-methoxymethylphenyl)(4,6-dimethoxypyrimidin-2-yl)ketone as yellow crystals (melting point: 100 to 101°C).

As described above, the amount of the iron powder used is large, and therefore, waste derived from iron increases. Further, acetic acid is used, and therefore, acidic wastewater is generated, and neutralization is required for disposal.

### Comparative Example 3 (X = H)

### {Method Described in Reference Example 2 (5), Patent Literature Description 1, WO 00/06553 (Aforementioned Patent Literature 1), pp. 17 to 18}

### Production of (2-amino-3-methoxymethylphenyl)(4,6-dimethoxypyrimidin-2-yl)methanol

3.1 g (10 millimoles) of (2-amino-3-methoxymethylphenyl)(4,6-dimethoxypyrimidin-2-yl)ketone was dissolved in 50 ml of a mixed solvent of tetrahydrofuran and water (volume ratio: 1:1). While the solution was stirred at room temperature, 0.6 g (16 millimoles) of sodium borohydride was added thereto, and stirring was further continued at room temperature for 2 hours. Then, 50 ml of ice water was added, and extraction was performed with ethyl acetate. The organic layer was washed with a saturated aqueous sodium chloride solution, and dried. The solvent was distilled off under reduced pressure, and the crystalline residue was washed with diisopropyl ether to obtain 2.8 g (yield: 92%) of (2-amino-3-methoxymethylphenyl)(4,6-dimethoxypyrimidin-2-yl)methanol as a white powder (melting point: 40 to 42°C).

By continuously performing the above Comparative Examples 2 and 3, (2-amino-3-methoxymethylphenyl)(4,6-dimethoxypyrimidin-2-yl)methanol which is included in the target compounds in the production method according to the present application was obtained. However, the yield when Comparative Examples 2 and 3 are continuously performed is 73.6%, which falls short of, for example, 84.6%, which is the yield when the above Examples 1 and 2 are continuously performed.

### Comparative Example 4

### Production of (2-amino-3-methoxymethylphenyl)(4,6-dimethoxypyrimidin-2-yl)methanol

16.7 g (0.05 mol) of (4,6-dimethoxypyrimidin-2-yl)(3-methoxymethyl-2-nitrophenyl)ketone, 22.3 g (0.40 mol, 8 equivalents) of an iron powder, and 50 ml of ethanol were added to a 100 ml four-necked flask equipped with a stirrer, a reflux condenser, a thermometer, and a dropping funnel, and the mixture was heated to 80°C with stirring. 50 ml (0.86 mol, 17.2 equivalents) of acetic acid was added dropwise therein, and the mixture was stirred at 80°C for 1 hour. The reaction liquid was cooled to 25°C, and then filtered using Celite. The filtrate containing iron acetate generated in the reaction was neutralized with 25% sodium hydroxide, and the precipitated iron hydroxide was filtered off. A time as long as 17 hours was required for the filtration of the iron hydroxide. Ethanol was distilled off from the filtrate under reduced pressure, and then, 50 ml of chlorobenzene and 50 ml of water were added, and the mixture was stirred and separated. The chlorobenzene layer was washed with 50 ml of water to obtain (2-amino-3-methoxymethylphenyl)(4,6-dimethoxypyrimidin-2-yl)methanol as a yellow chlorobenzene solution. This solution was analyzed by the HPLC absolute calibration curve method, and as a result, the yield was 50%.

In the production method shown in the above Comparative Example 4, when (2-amino-3-methoxymethylphenyl)(4,6-dimethoxypyrimidin-2-yl)methanol is produced from (4,6-dimethoxypyrimidin-2-yl)(3-methoxymethyl-2-nitrophenyl)ketone, the nitro group and the carbonyl group are reduced in one step. In this production method, a large amount of an iron powder and a large amount of acetic acid are used, and therefore, this production method is not economical, and increases waste. In addition, acetic acid is used, and therefore, neutralization is required. Further, the filterability of iron hydroxide generated in the neutralization is very low, and not only a long time is required for filtering off, but also the yield is poor. Therefore, the production method using a large amount of an iron powder and a large amount of acetic acid is not preferable for implementation on an industrial scale.

### Comparative Example 5

### 1. Production of (2-amino-5-chloro-3-methoxymethylphenyl)(4,6-dimethoxypyrimidin-2-yl)methanol

7.36 g (20 mmol) of (5-chloro-3-methoxymethyl-2-nitrophenyl)(4,6-dimethoxypyrimidin-2-yl)ketone, 8.93 g (160 mmol, 8 equivalents) of an iron powder, 20 ml of ethanol, and 20 ml (349 mmol, 17.5 equivalents) of acetic acid were added to a 100 ml four-necked flask equipped with a mechanical stirrer, a thermometer, and a reflux condenser, and the reaction system was heated to 50°C. After heat generation subsided, the system was stirred at a temperature of 75 to 80°C for 2 hours. The reaction system was cooled to room temperature, and then, 100 ml of water and 100 ml of toluene were added to the reaction system. The mixture was stirred for 10 minutes, and then filtered using Celite. The filtrate was separated, and the aqueous layer was reextracted with 100 ml of toluene. The toluene layers were combined, washed with water and then with a saturated aqueous sodium chloride solution, and dried over anhydrous sodium sulfate. Then, toluene was distilled off under reduced pressure to obtain a brown oil. 50 ml of toluene and 3.4 g of silica gel were added to this oil, and the mixture was stirred for 10 minutes, and then filtered. Toluene was distilled off from the filtrate under reduced pressure to obtain 6.4 g of a pale yellow oil. The crude yield was 94.2%. The HPLC purity of this crude oil was 93.9%. This crude oil was purified by silica gel column chromatography (ethyl acetate-hexane), and further recrystallized from isopropyl alcohol to obtain 4.7 g of white crystals of (2-amino-5-chloro-3-methoxymethylphenyl)(4,6-dimethoxypyrimidin-2-yl)methanol with a yield of 69.3%. (HPLC purity: 98.9%) Melting point: 80 to 82°C
¹H-NMR (300 MHz, CDCl₃, δ): 7.20 (d, J = 2.7 Hz, 1H), 6.99 (d, J = 2.7 Hz, 1H), 5.95 (s, 1H), 5.79 (d, J = 5.4 Hz, 1H), 5.18 (br, 2H), 4.73 (d, J = 5.4 Hz, 1H), 4.46 (dd, J = 15.2, 12.3 Hz, 2H), 3.95 (s, 6H), 3.31 (s, 3H) ppm.
GC-MS (m/z) : M⁺ = 340.1

### 2. Production of (2-amino-3-methoxymethylphenyl)(4,6-dimethoxypyrimidin-2-yl)methanol

6.4 g (18.8 mmol) of the crude oil of (2-amino-5-chloro-3-methoxymethylphenyl)(4,6-dimethoxypyrimidin-2-yl)methanol, 2.38 g (37.6 mmol) of ammonium formate, and 19 ml of methanol were added to a 50 ml eggplant-shaped flask equipped with a magnet stirrer and a dropping funnel, and the reaction system was purged with nitrogen. Then, 320 mg of 10% palladium on carbon (manufactured by Aldrich) was added, and the mixture was stirred at 40°C for 3 hours. After the completion of the reaction, the reaction system was cooled to room temperature, and filtered. Methanol was recovered under reduced pressure, and then, 30 ml of ethyl acetate and 50 ml of water were added to the reaction system, and the mixture was separated. The aqueous layer was reextracted with 30 ml of ethyl acetate. The ethyl acetate layer was washed with water and then with a saturated aqueous sodium chloride solution, and dried over anhydrous sodium sulfate. Then, ethyl acetate was distilled off under reduced pressure to obtain (2-amino-3-methoxymethylphenyl)(4,6-dimethoxypyrimidin-2-yl)methanol as 5.68 g of pale yellow oil with a yield of 99.0%. (HPLC purity: 96.0%)
¹H-NMR (300 MHz, CDCl₃, δ): 7.30 (dd, J = 7.5, 1.5 Hz, 1H), 7.01 (dd, J = 7.5, 1.5 Hz, 1H), 6.71 (dd, J = 7.5, 7.5 Hz, 1H), 5.93 (s, 1H), 5.84 (br, 1H), 5.16 (br, 2H), 4.51 (dd, J = 16.2, 12.0 Hz, 2H), 3.94 (s, 6H), 3.32 (s, 3H) ppm.
GC-MS (m/z): M⁺ = 305.

The production method shown in Comparative Example 5 is to obtain (2-amino-3-methoxymethylphenyl)(4,6-dimethoxypyrimidin-2-yl)methanol by reducing two functional groups, i.e., the nitro group and carbonyl group of (5-chloro-3-methoxymethyl-2-nitrophenyl)(4,6-dimethoxypyrimidin-2-yl)ketone, in one step, and then reducing the halogen atom.

The production method is an excellent production method that can solve the drawback of requiring high hydrogen pressure in the production method in which three functional groups, i.e., the chlorine atom, the nitro group, and the carbonyl group, are reduced in one step, described in the Patent Literature 2. However, also in this production method, a large amount of an iron powder and a large amount of acetic acid are used, and therefore, in industrial implementation, the operation of the working-up is complicated and moreover requires a long time, and further, the amount of waste is large, and the yield is relatively low. Therefore, further improvement has been desired.

### (HPLC Analysis Method)

Regarding the details of the above-described HPLC analysis method, the following literatures can be referred to, as required.
(a): The Chemical Society of Japan ed., "Shin Jikkenkagaku Koza (New Experimental Chemistry Course) 9 Bunsekikagaku (Analytical Chemistry) II", pp. 86 to 112 (1977), published by Shingo Iizumi, Maruzen Company, Limited (For example, regarding packing material - mobile phase combinations that can be used in the column, pp. 93 to 96 can be referred to.)
(b) The Chemical Society of Japan ed., "Jikkenkagaku Koza (Experimental Chemistry Course) 20-1 Bunsekikagaku (Analytical Chemistry) ", 5th ed., pp. 130 to 151 (2007), published by Seishiro Murata, Maruzen Company, Limited (For example, regarding the specific usage and conditions of reversed phase chromatography analysis, pp. 135 to 137 can be referred to.)

### (pH Measurement Method)

The pH was measured by a glass electrode type hydrogen ion concentration indicator. As the glass electrode type hydrogen ion concentration indicator, specifically, for example, model: HM-20P manufactured by DKK-TOA CORPORATION can be used.

### Industrial Applicability

The present invention provides a novel industrial production method for an aminophenylpyrimidinyl alcohol derivative represented by general formula (3), and a useful production intermediate.

In other words, according to the present invention, a nitrophenyl pyrimidinyl ketone derivative represented by general formula (1), which is easily available, can be used as a raw material, and the target aminophenylpyrimidinyl alcohol derivative can be efficiently produced with high yield, by a simple operation and under mild conditions, without using a special reaction apparatus. Further, the amount of waste is reduced, and the properties of the waste are preferable, which are also environmentally friendly.

The obtained aminophenylpyrimidinyl alcohol derivative represented by general formula (3) is a compound useful as an intermediate for the synthesis of a herbicide, and therefore, the present invention has a high industrial utility value.

## Claims

1. A method for producing an aminophenylpyrimidinyl alcohol derivative represented by general formula (3): 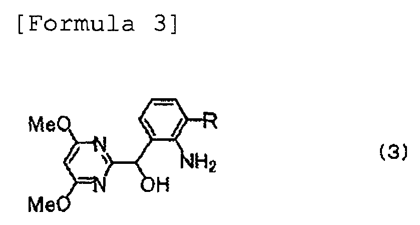 wherein R is an alkoxymethyl group,
comprising reducing a carbonyl group of a nitrophenyl pyrimidinyl ketone derivative represented by general formula (1): 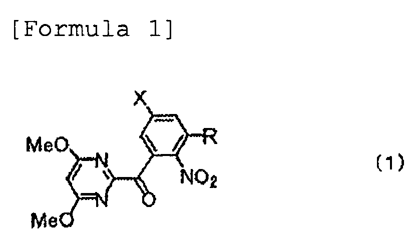 wherein X is a hydrogen atom, and R is the same as described above,
to produce a nitrophenylpyrimidinyl alcohol derivative represented by general formula (2): 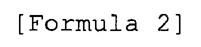 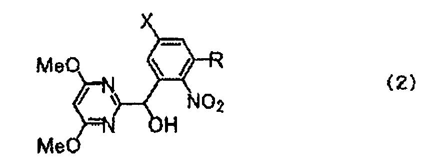 wherein X and R are the same as described above,
and then reducing the alcohol derivative using an iron powder in the presence of a salt selected from an ammonium salt and an alkaline-earth metal salt, without adding a free acid.

2. The method for producing an aminophenylpyrimidinyl alcohol derivative according to claim 1, wherein the nitrophenyl pyrimidinyl ketone derivative represented by general formula (1) is reduced using a metal hydride.

3. The method for producing an aminophenylpyrimidinyl alcohol derivative according to claim 2, wherein the nitrophenyl pyrimidinyl ketone derivative represented by general formula (1) is reduced using sodium borohydride.

4. The method for producing an aminophenylpyrimidinyl alcohol derivative according to claim 1, wherein the nitrophenylpyrimidinyl alcohol derivative represented by general formula (2) is reduced in the presence of an ammonium halide salt, an ammonium carbonate salt, an ammonium carboxylate salt, an alkaline-earth metal halide salt, or an alkaline-earth metal carbonate salt.

5. A nitrophenylpyrimidinyl alcohol derivative represented by general formula (2): 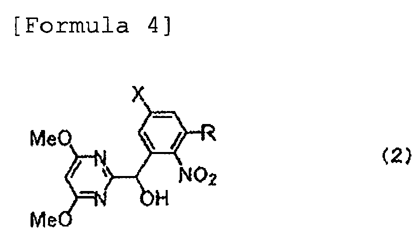 wherein X is a hydrogen atom, and R is an methoxymethyl group.

## Patentansprüche

1. Ein Verfahren zur Herstellung eines Aminophenylpyrimidinyl-Alkoholderivats, dargestellt durch die allgemeine Formel (3): 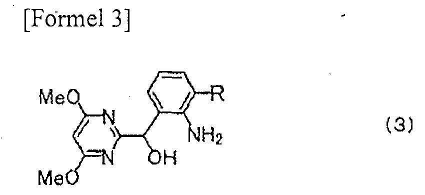 wobei R eine Alkoxymethylgruppe ist,
umfassend Reduzieren einer Carbonylgruppe eines Nitrophenylpyrimidinyl-Ketonderivats, dargestellt durch die allgemeine Formel (1): 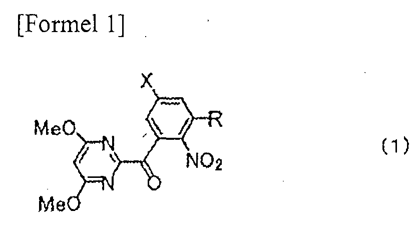 wobei X ein Wasserstoffatom ist und R wie vorstehend beschrieben ist,
um ein Nitrophenylpyrimidinyl-Alkoholderivat, dargestellt durch die allgemeine Formel (2), herzustellen: 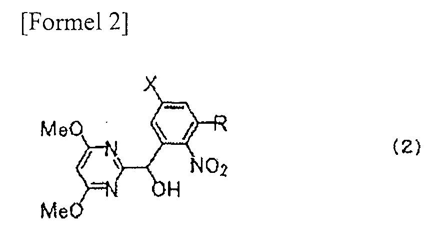 wobei X und R wie vorstehend beschrieben sind,
und dann Reduzieren des Alkoholderivats unter Verwendung eines Eisenpulvers in Gegenwart eines Salzes, ausgewählt aus einem Ammoniumsalz und einem Erdalkalimetallsalz, ohne eine freie Säure hinzuzufügen.

2. Das Verfahren zur Herstellung eines Aminophenylpyrimidinyl-Alkoholderivats nach Anspruch 1, wobei das Nitrophenylpyrimidinyl-Ketonderivat, dargestellt durch die allgemeine Formel (1), unter Verwendung eines Metallhydrids reduziert wird.

3. Das Verfahren zur Herstellung eines Aminophenylpyrimidinyl-Alkoholderivats nach Anspruch 2, wobei das Nitrophenylpyrimidinyl-Ketonderivat, dargestellt durch die allgemeine Formel (1), unter Verwendung von Natriumborhydrid reduziert wird.

4. Das Verfahren zur Herstellung eines Aminophenylpyrimidinyl-Alkoholderivats nach Anspruch 1, wobei das Nitrophenylpyrimidinyl-Alkoholderivat, dargestellt durch die allgemeine Formel (2), in Gegenwart eines Ammoniumhalogenidsalzes, eines Ammoniumcarbonatsalzes, eines Ammoniumcarboxylatsalzes, eines Erdalkalimetallhalogenidsalzes oder eines Erdalkalimetallcarbonatsalzes reduziert wird.

5. Ein Nitrophenylpyrimidinyl-Alkoholderivat, dargestellt durch die allgemeine Formel (2): 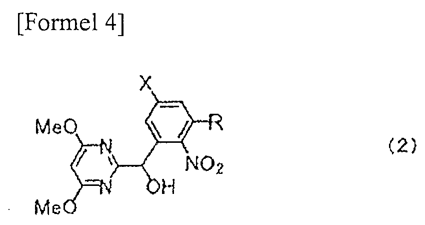 wobei X ein Wasserstoffatom ist und R eine Methoxymethylgruppe ist.

## Revendications

1. Méthode de production d'un dérivé d'alcool aminophénylpyrimidinylique représenté par la formule générale (3) : 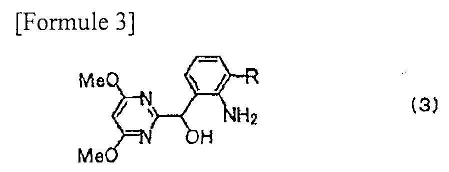 dans laquelle R est un groupe alcoxyméthyle,
comprenant la réduction d'un groupe carbonyle d'un dérivé de nitrophénylpyrimidinylcétone représenté par la formule générale (1) : 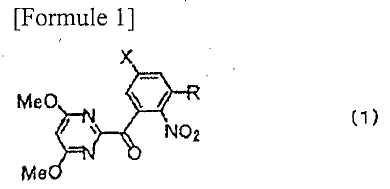 dans laquelle X est un atome d'hydrogène, et R est tel que décrit ci-dessus,
pour produire un dérivé d'alcool nitrophénylpyrimidinylique représenté par la formule générale (2) : 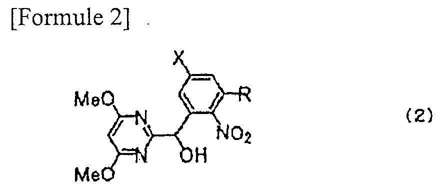 dans laquelle X et R sont tels que décrits ci-dessus,
puis la réduction du dérivé d'alcool à l'aide de poudre de fer en présence d'un sel choisi parmi un sel d'ammonium et un sel de métal alcalino-terreux, sans ajout d'acide libre.

2. Méthode de production d'un dérivé d'alcool aminophénylpyrimidinylique selon la revendication 1, dans laquelle le dérivé de nitrophénylpyrimidinylcétone représenté par la formule générale (1) est réduit à l'aide d'un hydrure métallique.

3. Méthode de production d'un dérivé d'alcool aminophénylpyrimidinylique selon la revendication 2, dans laquelle le dérivé de nitrophénylpyrimidinylcétone représenté par la formule générale (1) est réduit à l'aide de borohydrure de sodium.

4. Méthode de production d'un dérivé d'alcool aminophénylpyrimidinylique selon la revendication 1, dans laquelle le dérivé d'alcool nitrophénylpyrimidinylique représenté par la formule générale (2) est réduit en présence d'un sel d'halogénure d'ammonium, d'un sel de carbonate d'ammonium, d'un sel de carboxylate d'ammonium, d'un sel d'halogénure de métal alcalino-terreux, ou d'un sel de carbonate de métal alcalino-terreux.

5. Dérivé d'alcool nitrophénylpyrimidinylique représenté par la formule générale (2) : 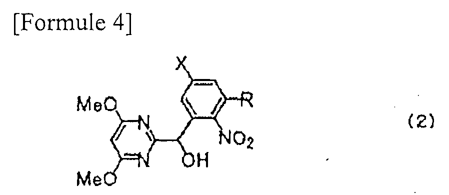 dans laquelle X est un atome d'hydrogène, et R est un groupe méthoxyméthyle.
